# EUROPEAN PATENT SPECIFICATION

(11) **EP 2 414 511 B1**
(45) Date of publication and mention of the grant of the patent: **26.07.2017**
(21) Application number: 09842838.6
(22) Date of filing: 19.06.2009
(51) Int. Cl.: C12N 5/0789, C12N 5/02, C12N 5/071

(54) **ISOLATION OF HUMAN UMBILICAL CORD BLOOD-DERIVED MESENCHYMAL STEM CELLS**
ISOLIERUNG AUS MENSCHLICHEM NABELSCHNURBLUT STAMMENDER MENSENCHYMALER STAMMZELLEN
ISOLEMENT DE CELLULES SOUCHES MÉSENCHYMATEUSES ISSUES DE SANG DE CORDON OMBILICAL HUMAIN

(30) Priority: 31.03.2009 US 165193 P
(43) Date of publication of application: 08.02.2012
(73) Proprietor: The Board of Regents of The University of Texas System, Austin, TX 78701 (US)
(72) Inventor: CHEN, Xiao-Dong, San Antonio, TX 78229 (US); LU, Zhongding, San Antonio, TX 78229 (US)
(74) Representative: Icosa
(86) International application number: PCT/US2009/047981
(87) International publication number: WO 2010/114572

(56) References cited:
- US-A1- 2007 128 722
- US-A1- 2009 081 784
- US-A1- 2009 081 784
- XIAO-DONG CHEN ET AL: "Extracellular matrix made by bone marrow cells facilitates expansion of marrow-derived mesenchymal progenitor cells and prevents their differentiation into osteoblasts", JOURNAL OF BONE AND MINERAL RESEARCH, WILEY-BLACKWELL PUBLISHING, INC, NEW YORK, NY, US, vol. 22, no. 12, 1 December 2007 (2007-12-01), pages 1943-1956, XP002634940, ISSN: 0884-0431, DOI: 10.1359/JBMR.070725 [retrieved on 2007-08-06]
- LEE M W ET AL: "Mesenchymal stem cells from cryopreserved human umbilical cord blood", BIOCHEMICAL AND BIOPHYSICAL RESEARCH COMMUNICATIONS, ACADEMIC PRESS INC. ORLANDO, FL, US, vol. 320, no. 1, 16 July 2004 (2004-07-16) , pages 273-278, XP004519095, ISSN: 0006-291X, DOI: 10.1016/J.BBRC.2004.04.206
- KOCH THOMAS G ET AL: "Isolation of mesenchymal stem cells from equine umbilical cord blood", BMC BIOTECHNOLOGY, BIOMED CENTRAL LTD. LONDON, GB, vol. 7, no. 1, 30 May 2007 (2007-05-30), page 26, XP021029466, ISSN: 1472-6750, DOI: 10.1186/1472-6750-7-26
- GAO FENG ET AL.: 'Extracellular matrix gel is necessary for in vitro cultivation of insulin producing cells from human umbilical cord blood derived mesenchymal stem cells.' CHINESE MEDICAL JOURNAL. vol. 121, no. 9, May 2008, pages 811 - 818, XP008154606
- DARKO BOSNAKOVSKI ET AL.: 'Chondrogenic differentiation of bovine bone marrow mesenchymal stem cells (MSCs) in different hydrogels: influence of collagen type II extracellular matrix on MSC chondrogenesis.' BIOTECHNOLOGY AND BIOENGINEERING. vol. 93, no. 6, April 2006, pages 1152 - 1163, XP002495753
- MYOUNG WOO LEE ET AL.: 'Mesenchymal stem cells from cryopreserved human umbilical cord blood.' BIOCHEMICAL AND BIOPHYSICAL RESEARCH COMMUNICATIONS. vol. 320, no. 1, July 2004, pages 273 - 278, XP004519095
- THOMAS G. KOCH ET AL.: 'Isolation of mesenchymal stem cells from equine umbilical cord blood.' BMC BIOTECHNOLOGY. vol. 7, May 2007, page 26, XP021029466
- YUICHI HORI ET AL.: 'Differentiation of insulin-producing cells from human neural progenitor cells.' PLOS MEDICINE. vol. 2, no. 4, April 2005, page E103, XP002451913
- SIAM OOTTAMASATHIEN ET AL.: 'Directed differentiation of embryonic stem cells into bladder tissue.' DEVELOPMENTAL BIOLOGY. vol. 304, no. 2, April 2007, pages 556 - 566, XP022019946

## Description

### BACKGROUND OF THE INVENTION

The present application claims benefit of priority to U.S. Provisional Application Serial No. 61/165,193 filed March 31, 2009.

This invention was made with government support under 5R21AG25466-2 awarded by the National Institutes of Health. The government has certain rights in the invention.

### 1. Field of the Invention

The present invention relates generally to the field of biology. More particularly, it relates to methods for isolating and growing mesenchymal stem cells from umbilical cord blood (UCB).

### 2. Description of the Related Art

Stem cells are one of the most fascinating areas of biomedicine today. Mesenchymal stem cells (MSC) are of great therapeutic potential due to their capacity of self-renewal and multilineage differentiation.

Recently, umbilical cord blood (UCB) has been proposed as an alternative source of mesenchymal stem cells (MSCs) for stem cell therapy in areas of arthritis, heart disease, nerve, and tissue regeneration. The advantages of using UCB-MSCs over bone marrow-derived MSCs (BM-MSCs) are 1) UCB is abundantly available, and can be harvested without harm to the donors; and 2) UCB-MSCs have higher expansion potential and greater differentiation capability. However, the major limitation in the use of UCB-MSCs for both research and clinical applications is that the frequency of MSCs in UCB is extremely low (∼0.4 to 30 out of 1 X 10⁸ mononuclear cells, MNCs) and the successful rate of UCB-MSCs isolation is also low (∼ 30%). As stem cells, it is difficult to expand them in long-term culture without the loss of their stem cell properties. To date, MSCs are isolated from bone marrow or any other tissues by the classic plastic adhesion method because of a lack of specific markers that can define these cells. Wolfe *et al.,* 2008; Soleimani and Nadri, 2009; Kern *et al.,* 2006. Comparative analysis of mesenchymal stem cells from bone marrow, umbilical cord blood, or adipose tissue. Stem Cells 24:1294-1301.

Using the same procedure, most of the extremely immature MSCs in UCB are likely missed because their ability to adhere to plastic is poor. Recently, it was reported that extracellular matrix (ECM) made by bone marrow cells enhanced MSC attachment and proliferation, and retained their stem cell properties. Chen et al., 2007, JBMR, 22:1943.

The US patent application 2007/128722 discloses methods for proliferating human mesenchymal stem cells comprising isolating MSCs from human cord blood (CB-MSCs) then cultured on dishes covered with ECM prepared from human foreskin fibroblasts, from human placenta, from Matrigel or from bovine corneal endothelial cells.

Here, the invention provides methods for isolating, promoting proliferation of MSCs by adherence to ECMs formed by culturing human bone marrow cells. Using this system, a large number of hUCB-MSCs can be isolated, indicating that the frequency is at least 1000-fold greater than previously reported by others who isolated UCB-MSCs with the classic, fibronectin, or FBS pre-coated plastic adhesion method. More importantly, MSCs obtained by the ECM formed embryonic bodies *in vitro,* a unique feature of embryonic stem cells, and generated tissues originated from 3 embryonic germ layers *in vivo.*

### SUMMARY OF THE INVENTION

The present invention provides methods for isolating mesenchymal stem cells (MSCs) from umbilical cord blood by adherence to an extracellular matrix (ECM) and uses for the isolated MSCs.

In one aspect, the invention provides a method of isolating mesenchymal stem cells (MSCs) comprising (a) collecting a sample; (b) seeding the sample on an extracellular matrix (ECM) formed by culturing human bone marrow cells-precoated culture dish; and (c) isolating the MSCs. The step (a) of the method further comprises centrifuging the sample to collect mononuclear cells before seeding.

In another aspect, the invention provides a method which comprises implanting the isolated MSCs to obtain differentiated tissue. In other embodiments, the method further comprises implanting the isolated MSCs to obtain differentiated tissue.

In other aspects, the invention provides a method of promoting MSC proliferation comprising (a) obtaining a sample of isolated MSCs; and (b) seeding the isolated MSCs onto an ECM formed by culturing human bone marrow cells to promote proliferation of the isolated MSC sample.

In still further aspects, the invention provides a method of producing differentiated tissues comprising (a) obtaining a sample of isolated MSCs; and (b) implanting the isolated MSCs into an immunocompromised mouse to obtain differentiated tissue.

The sample may be from any tissue or sample that contains MSCs. In particular embodiments, the sample is umbilical cord blood (UCB). The sample may be in particular embodiments from a human. In particular embodiments, the sample is collected after birth.

The extracellular matrix is derived from human bone marrow cells. In particular embodiments, the ECM may comprise collagen type I, collagen type III, fibronectin, biglycan, decorin, perlecan, and/or laminin.

The differentiated tissue may comprise a number of layers. In particular embodiments, the differentiated tissue comprises three embryonic germ layers-derived tissues. In some embodiments, the differentiated tissue comprises endoderm-gland; mesoderm-bone, muscle, fat, blood vessel; and/or ectoderm-nerve fiber.

The embodiments in the Example section are understood to be embodiments of the invention that are applicable to all aspects of the invention.

The use of the term "or" in the claims is used to mean "and/or" unless explicitly indicated to refer to alternatives only or the alternatives are mutually exclusive, although the disclosure supports a definition that refers to only alternatives and "and/or".

Throughout this application, the term "about" is used to indicate that a value includes the standard deviation of error for the device or method being employed to determine the value.

Following long-standing patent law, the words "a" and "an," when used in conjunction with the word "comprising" in the claims or specification, denotes one or more, unless specifically noted.

The term "therapeutically effective" as used herein refers to an amount of cells and/or therapeutic composition (such as a therapeutic polynucleotide and/or therapeutic polypeptide) that is employed in methods of the present invention to achieve a therapeutic effect, such as wherein at least one symptom of a condition being treated is at least ameliorated.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### BRIEF DESCRIPTION OF THE FIGURES

The following drawings form part of the present specification and are included to further demonstrate certain aspects of the present invention. The invention may be better understood by reference to one or more of these drawings in combination with the detailed description of specific embodiments presented herein.

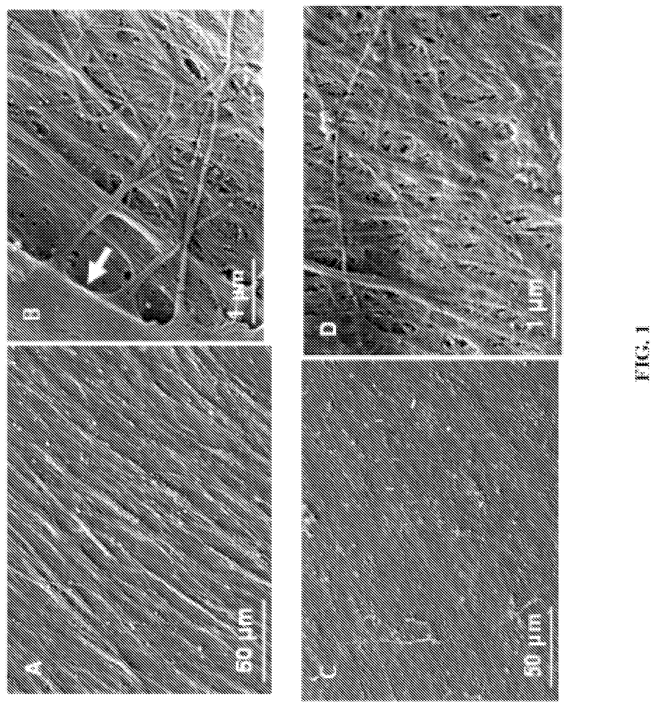

**FIGS. 1A-D** Scanning micrographs of human stromal cell cultures and their ECM. SEM micrographs of cultured stromal cells before (FIGS. 1A, B), and after cell removal (FIGS. 1C, D). The arrow in panel B denotes a cell. A standard procedure based on the inventors' previous studies was utilized. Stromal cells from passages 1 or 2 were seeded onto tissue culture plastic at 1 x 10⁴ cells/cm2, and cultured for 15 days. The medium (α-MEM containing 15% FBS) was changed every 3-4 days, and ascorbic acid (50 µM) was added during the final 8 days of culture. After extensive washing with PBS, cells were removed by incubation with 0.5% Triton X-100 containing 20 mM NH4OH in PBS for 5 minutes at room temperature. After washing with PBS 4 times, PBS containing 50 µg/ml gentamicin and 0.25 µg/ml fungizone was added to the plates, which were stored at 4°C for up to 4 months.

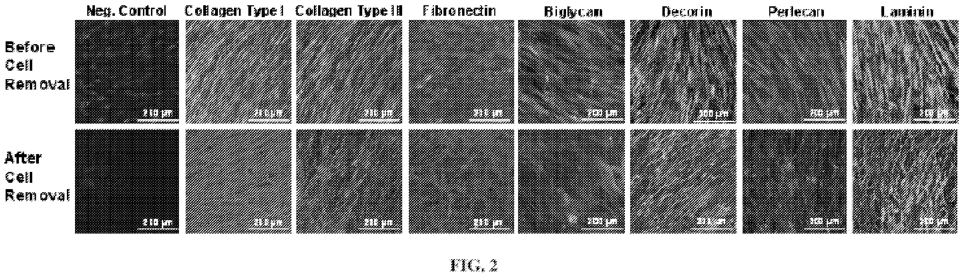

**FIG. 2** Confocal images showing localization of collagen type I, III, fibronectin, biglycan, decorin, perlecan, and laminin in the ECM elaborated by human bone marrow stromal cells. Proteins were detected using antibodies against the indicated components and green fluorescent-labeled secondary antibodies. Nonspecific isotype IgG was used as a negative control (Neg. Control). Nuclear staining with DAPI is shown in blue.

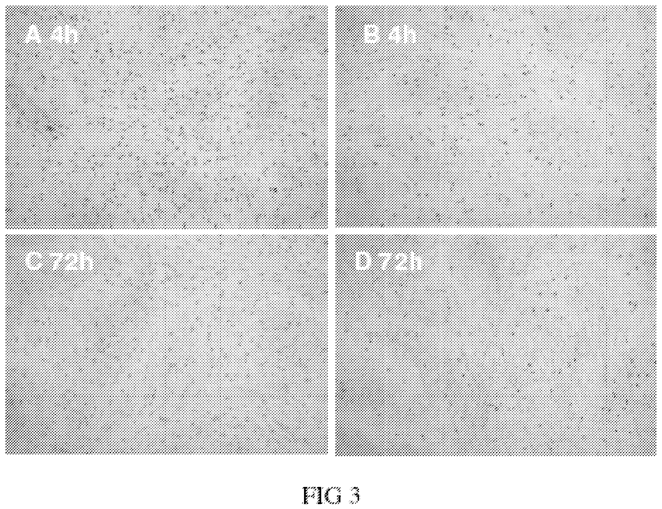

**FIGS. 3A-D** Non-adherent cells removed from 2D and ECM plate 4 h and 72 h after primary seeding were reseeded onto ECM plates. 24 h after reseeding, non-adherent cells from the primary 2D plate showed 5 times more cells attached (FIGS. 3A, C; crystal violet stain) than from the primary ECM plate (FIGS. 3B, D).

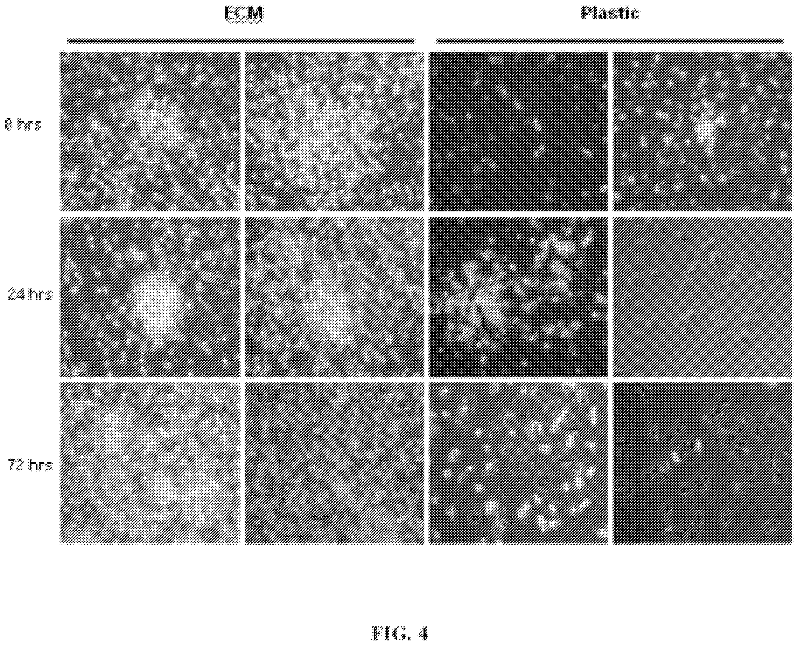

**FIG. 4** The ECM facilitates UCB-derived MSCs attachment and expansion. Human UCB was purchased from Texas Cord Blood Bank (San Antonio, TX). Mononuclear cells (MNC) isolated from UCB were seeded onto the ECM or uncoated plastic at 1 x 10⁶ MNC/cm² and incubated for the various times indicated at 37°C. Then, non-adherent cells were removed by washing with PBS. Original magnification, x 100.

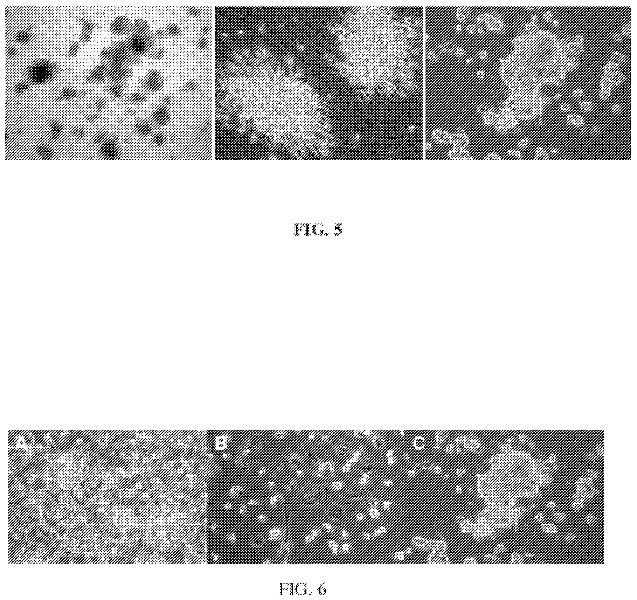

**FIG. 5** Colony formation. UCB-MSCs isolated by the ECM adhesion formed numerous colonies (left panel, original magnification, x 50, and middle panel, original magnification, x 200), and some of these generated embryonic bodies (right panel, original magnification, x 200).
**FIGS. 6A-C** Colony formation. UCB-MSCs were seeded onto the ECM (FIG. 6A) or uncoated plastic (FIG. 6B) at 1 x 10⁶ MNC/cm² and incubated for 72 hours at 37°C (original magnification, x 100). (FIG. 6C) Embryonic-like bodies formed on ECM coated plates (original magnification, x 200).

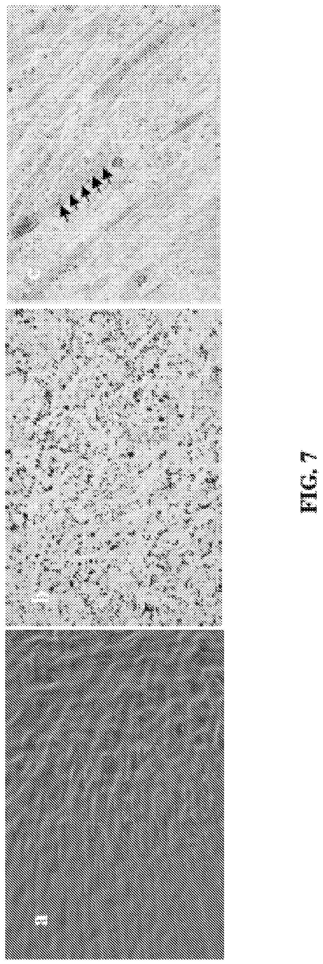

**FIGS. 7A-C** Cell Differentiation. (FIG. 7A) Undifferentiated UCB-MSCs. (FIG. 7B) UCB-MSC adipogenesis, oil red stain showed the lipid droplets. (FIG. 7C) UCB-MSC myogenesis, hematoxylin staining showed myotube with multiple nuclei (arrows).

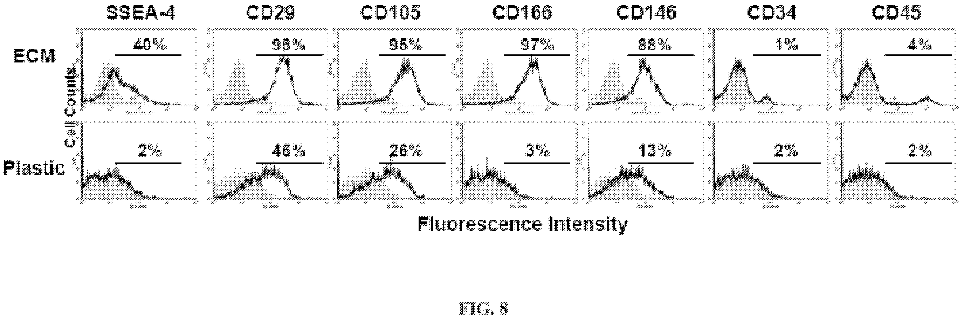

**FIG. 8** Flow cytometric analysis of cells isolated by the ECM adhesion (ECM) vs. cells isolated by a classical plastic adhesion method (Plastic). In the same experiments previously described in FIG. 4, single-cell suspensions were obtained from cell incubation on the ECM or plastic for 72 hrs after treatment with trypsin, and stained with the various primary antibodies and FITC-conjugated secondary antibodies. Cells stained with primary non-specific antibody (isotype, IgG) were serviced as negative control (gray-peaks). The stained cells were analyzed using a Becton Dickinson FACStarplus flow cytometer with 10,000 events, collected for each sample.

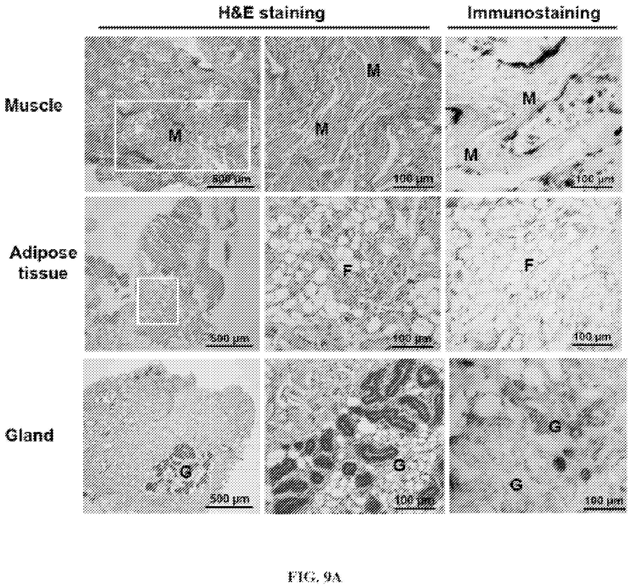

**FIGS. 9A-B** UCB-MSCs isolated by the ECM generated tissues originated from 3 embryonic germ layers *in vivo.* UCB-MSCs isolated by the ECM and continuously expanded on the ECM or UCB-MSCs isolated by plastic and continuously expanded on plastic were loaded onto Gelfoam or hydroxyapatite /tricalcium phosphate (HA/TCP) that favorably induces skeletogenesis, and implanted subcutaneously into the dorsal surface of 10-wk-old immunodeficient beige mice. Each vehicle was loaded with 0.5 x 10⁶ cells. Three implantations were performed for each condition. Implants were harvested after 8 wks of implantation and processed for histological analysis. The sections were stained with H&E. In addition, Bielschowsky's silver staining was used to specifically identify nerve (see middle panel of Nerve fibers). To determine the origin of generated tissue, a section adjacent to the H&E stained section was stained with an antibody specifically against human nuclear ribonucleoprotein purchased from Millipore (Billerica, MA). Mouse and human tissues served as negative and positive controls, respectively. Skeletal tissue generated in ossicles has been defined as from donor origin (30). A, artery; B, bone; C, capillary; E, endothelial cells; F, fat; G, gland; M, muscle; and N, nerve.

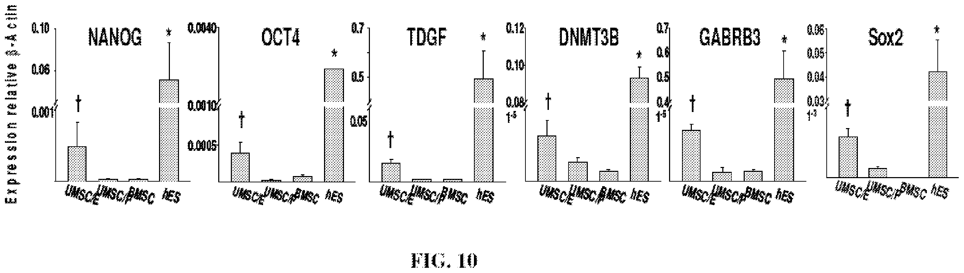

**FIG. 10** Gene expression profiles of UCB cells isolated by the ECM adhesion method. RNA was prepared from UCB cells (passage 1) pre-isolated and maintained on the ECM (UMSC/E) or on plastic (UMSC/P) separately from 4 individual donors. The transcripts of interest were determined by real-time PCR using TaqMan PCR Master Mix and Assay Demand (Applied Biosystems). RNA isolated from human ES cells [(hES) cell line H7] was kindly provided by Dr. Christopher Navara from UTSA. RNA for human MSCs (BMSC) was prepared from human bone marrow cells purchased from ALLCELLS (Emeryville, CA) as described in Method. **P* < 0.01 (n = 4), hES vs. UMSC/E, or UMSC/P, or BMSC. ^{†}*P* < 0.01 (n = 4) UMSC/E vs. UMSC/P, or BMSC.

### DESCRIPTION OF THE ILLUSTRATIVE EMBODIMENTS

The present invention provides methods for isolating MSCs by adherence to an ECM and uses for the isolated MSCs.

### I. Mesenchymal stem cells (MSCs)

MSCs are of great therapeutic potential due to their capacity of self-renewal and multilineage differentiation. MSCs are multipotent stem cells that can differentiate into a variety of cell types. Cell types that MSCs have been shown to differentiate into *in vitro* or *in vivo* include osteoblasts, chondrocytes, myocytes, and adipocytes. The term "stem cell" as used herein refers to a cell that gives rise to one or more lineages of cells.

It has been reported that MSCs could be isolated from various tissues, including periosteum, trabecular bone, adipose tissue, synovium, skeletal muscle, deciduous teeth, fetal pancreas, lung, liver, amniotic fluid, cord blood and umbilical cord tissues. Among those, cord blood may be the ideal sources due to their accessibility, painless procedures to donors, promising sources for autologous cell therapy and lower risk of viral contamination. Umbilical cord blood stem cells can be obtained from the umbilical cord immediately after birth. These stem cells are less mature than those stem cells found in the bone marrow of adults or children. Moreover, human umbilical cord blood (hUCB) contains mesenchymal stem cells MSCs that have higher expansion potential and greater differentiation capability than adult marrow-derived MSCs (BM-MSCs). However, these cells have been difficult to obtain with traditional methods because the success rate of UCB-MSC isolation is low. With traditional methods, for any given 10 UCB samples, UCB-MSCs can be isolated from only 3 samples (a 30% success rate), and in these samples in which UCB-MSCs can be found, the absolute number of MSCs in UCB is extremely low (∼5 to 30 out of 1 x 10⁸ mononuclear cells, MNCs).

### II. Isolation of MSCs on the ECM

To date, the isolation of MSCs has depended upon their plastic-adhesion capacity. Thus, some true MSCs in UCB are likely missed because their ability to adhere to plastic is poor. Furthermore, stem cells require a specialized microenvironment to maintain their function. Current tissue culture technology does not provide this environment as evidenced by loss of MSC stem cell properties. Instead, they undergo senescence, "spontaneously" commit to a particular cell lineage, or become to transformed cells.

In some aspects, the invention provides for the isolation of MSCs by adherence to an ECM. By using the ECM adhesion procedure, isolation of a surprisingly large number of embryonic-like stem cells from human umbilical cord blood was achieved. With the currently described method, a cell-free native ECM made by human bone marrow (hBM) cells can be reconstituted. The ECM comprises, at least in part, collagen type I and III, fibronectin, small leucine-rich proteoglycans such as biglycan and decorin, and major components of basement membrane such as a perlecan and laminin. All these matrix proteins are very important in regulating cell adhesion, migration, proliferation, differentiation and survival. The components and unique structure of hBM-ECM mimic key features of microenvironment *in vivo* that supports MSCs, and remarkably enhance hUCB-MSC attachment and proliferation, and retain their stem cell properties.

The ECM made by bone marrow cells enhanced UCB-MSC attachment and proliferation, and retention of their stem cell properties. Using this system, it was found that human UCB contains a large number of MSCs that adhere to ECM, but not to plastic. Furthermore, numerous colonies were formed when primary cells were cultured on the ECM with a low seeding density (3 x 10⁴ - 1 x 10⁵ MNCs/cm²). This data indicates that UCB-MSCs cultured on the EMC have much greater colonogenic capability than on plastic. According to the measurement of colony formation units (CFUs) the frequency of UCB-MSCs is 22,800 - 37,000 out of 1 x 10⁸ MNCs, at least 1,000-fold (760 - 92,500) greater than previously reported by others (0.4 - 30 out of 1 x 10⁸ MNCs).

More importantly, the studies indicated that without any of specific differentiation induction the implantation of hUCB-derived MSCs (hUCB-MSCs) obtained by ECM into immunocompromised mice generated 3 embryonic germ layers-derived tissues including bone, muscle, fat, blood vessel, gland, and nerve fiber. Since the hUCB-MSCs isolated by ECM and subsequently cultured on ECM are native and pluripotential exhibiting hES cell characteristics, these cells were named "human umbilical cord blood derived embryonic-like stem cells (hUCB-ELSCs).

Using this novel technology, a surprisingly large number of UCB-ELSCs can be obtained from only one cord blood unit. Without any of specific differentiation induction the hUCB-MSCs cultured on ECM, even at passage 6-8, could be differentiated very effectively toward various tissues originated from 3 embryonic germ layers *in vivo* like hES. Based on these unique features, the currently disclosed method is very useful for:
**1. Tissue engineering basic research:** A large number of highly purified hUCB-MSCs obtained by the ECM adhesion will facilitate to study molecular mechanisms that control their behavior.
**2. Drug discovery:** Highly purified hUCB-MSCs maintained on the ECM can be used for drug screening *in vitro.*
**3. Tissue engineering clinical uses:** A large numbers of native and multipotential UCB-ELSCs may be substitutes of ES cells for cell-based clinical applications in the foreseeable future.

### III. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1 - PREPARATION OF CELL-FREE ECM FROM CULTURED

### BONE MARROW CELLS

1) Purchase freshly isolated human bone marrow mononuclear cells (containing MSCs, obtained from 20-30-year-old donors) from ALLCELLS (Emeryville, CA).
2) Plate cells on T175 flask at a density of 8.5-17 x 10⁴ per cm², and culture in α-MEM containing glutamine (4 mM), penicillin (100 U/ml), streptomycin (100 µg/ml), and 15% pre-selected fetal bovine serum. After 24 h, remove non-adherent cells, add fresh medium, and grow to 70% confluence (2-3 weeks).
3) Wash cultures with PBS. Detach the adherent cells using 0.05% trypsin. Collect, resuspend cells and reseed onto tissue culture plastic at 6 x 10³ cells/cm², and culture for 15 days. Change half medium every 3-4 days; ascorbic acid (50 µM) were added during the final 8 days of culture. After extensive washing with PBS, remove cells by incubation of 0.5% Triton X-100 containing 20 mM NH₄OH in PBS, pH 7.4, for 5 minutes at 37°C. Then wash the plates with PBS 4 times, add PBS containing 50 µg/ml gentamicin and 0.25 µg/ml fungizone, and store at 4°C up to 4 months.

### EXAMPLE 2 - ISOLATION AND CULTURE OF MSCS FROM HUCB

1) Isolation of hUCB mononuclear cells (MNC) using Ficoll density Gradient: Dilute the anticoagulated cord blood (1:1) with Balanced salt solution (BSS). Lay the diluted cord blood slowly on 10ml of Ficoll-Paque PREMIUM solution (GE Healthcare BioSciences Corp., Piscataway, NJ) layer (ratio 4:1) in a 50-ml tube. Centrifuge the layered blood samples at 480 g for 30 min at 18-20°C. Collect the mononuclear/white layer in a new 50 ml tube; add 3 volumes of BSS to the MNCs; centrifuge the cell suspension at 480 g for 6 min at 18-20°C; resuspend the pellet in 10 ml αMEM containing 2% FBS. Count the cells, and check the viability.
2) Seed at a density of 1 x 10⁶ MNC/cm2 into human bone marrow cell-derived ECM-precoated culture plates or dishes. Remove non-adherent cells 24 hours after initial plating. Wash adherent cells vigorously twice with PBS and shaking to remove any non-adherent cells containing hematopoietic cells, and add fresh medium. Cultivate the resulting fibroblastoid adherent cells containing MSCs (hUCB-MSCs) at 37°C in a humidified atmosphere containing 5% CO₂.
3) Change medium once a week. The expansion medium consists 20% FBS. Maintain MSCs in the medium until they reached 70% to 90% confluence. Cells can be harvested at subconfluence using 0.05% of Trypsin. At the second passage re-plate the cells at a mean density of 6 x 10³/cm².
4) Generation of single separated, fibroblastoid colonies termed fibroblastoid colony-forming units (CFU-F) can be achieved by initially seeding the MNCs at a low density (1 x 10³ to 1 x 10⁵ cells per cm²).

### EXAMPLE 3 - COMPARISON OF MSCs ADHERENCE TO ECM VS. PLASTIC

**Methods:** To compare the ability of MSCs to adhere to marrow-derived ECM versus plastic, cells were seeded at 1x10⁶ cells/cm² onto plastic and incubated for 4, 24, and 72 hours. The non-adherent cells were collected from the plastic and re-seeded onto the ECM and incubated for an additional 24 hours. The adherent cells were stained with crystal violet and quantified (FIG. 8). Adipogenesis and myogenesis were determined by cells maintained in adipogenic or myogenic medium, respectively.

**Results:** The most adherent cells in UCB were able to attach to the ECM as soon as 4 hrs of incubation. In contrast, fewer cells attached to plastic. However, non-adherent cells collected from plastic contained a large number of cells that attached to the ECM.

The cells adherent on the ECM can differentiate into adipocytes and muscle cells *in vitro.*

### EXAMPLE 4 - IMPLANTATION AND DIFFERENTIATION EVALUATION

1) Implantation: Load 0.5 x 10⁶ pre-cultured hUCB-MSCs (Passage 6-8) into a transplantation vehicle hydroxyapatite/tricalcium phosphate (HA/TCP) ceramic powder or Gelfoam and implanted subcutaneously into the dorsal surface of 10-week-old immunodeficient beige mice (NIH-bg-nu-xid).
2) Differentiation evaluation: Harvest and fix the implants with 4% formaldehyde at room temperature after implantation 2 - 4 months. Examine the serial 4-µm paraffin embedded implants by H&E and immunostaining. The tissue differentiated from donor hUCB-MSCs can be detected by immunostaining with anti-human specific RNP antibody (1:60; Millipore/Chemicon).

### EXAMPLE 5 - RESULTS

**A large number of UCB-MSCs adhered to the ECM, but not to plastic.** Extracellular matrix (ECM, FIGS. 1, 2) made by bone marrow cells enhanced hUCB-MSC attachment and proliferation, and retained their stem cell properties. Using this system, it was found that human UCB contains a large number of MSCs that adhere to the ECM, but not to plastic. Numerous colonies were formed when primary cells were cultured on the ECM with a seeding density 3 x 10⁴ - 1 x 10⁵ MNCs/cm² (FIGS. 3, 4). This data indicates that hUCB-MSCs cultured on the ECM have much greater colonogenic capability than on the plastic. According to the measurement of colony formation units (CFU) the frequency of hUCB-MSCs is 22,800 - 37,000 out of 1 x 10⁸ MNCs, at least 1,000 (760 - 92,500) fold greater than previously reported by others (0.4 - 30 out of 1 x 10⁸ MNCs).

**UCB cells adhered to the ECM expressed SSEA-4 and other MSC markers, but no hematopoietic cell markers.** The phenotypes of cells adhered to the ECM were determined by flow cytometric analysis, suggesting that ∼ 40% of these cells expressed a ES cell marker SSEA-4 (15), and ∼ 90% of the cells also expressed several MSC markers including CD29, CD105, CD166 and CD146, but no the expression of CD34 and CD45 hematopoietic cell markers (FIG. 8). In contrast, cells adhered to plastic contained fewer SSEA-4⁺ cells and small number of cells expressing those MSC markers. These results suggested that the phenotypes of cells adhered to the ECM were very different from those adhered to plastic. The cells isolated by the ECM adhesion are unique, which may contain a relatively homogenous population of MSCs. These cells may also have some characteristics of ES cells.

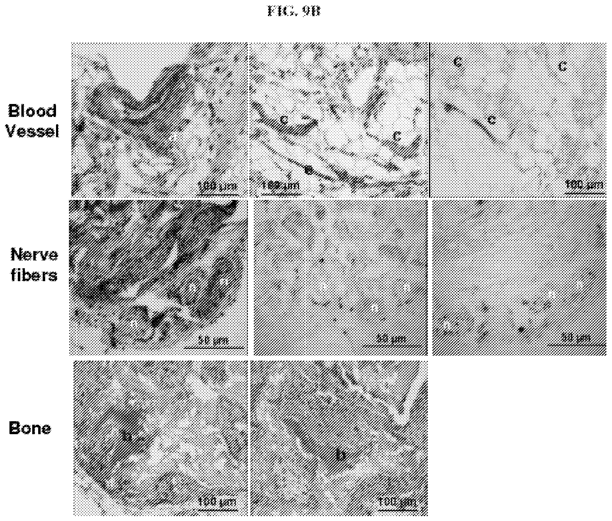

**hUCB-MSCs cultured on the ECM generated tissues originated from 3 embryonic germ layers *in vivo.*** ECM system enriches embryonic-like cells from human umbilical cord blood. hUCB-MSCs obtained by the ECM and subsequently cultured on ECM formed embryonic bodies *in vitro* (FIGS. 5 and 6), a unique feature of embryonic stem cells. Without any of specific differentiation induction, the implantation of hUCB-MSCs into immunocompromised mice generated 3 embryonic germ layers-derived tissues including: endoderm-gland; mesoderm-bone, muscle, fat, blood vessel; and ectoderm-nerve fiber. These tissues are differentiated from donor hUCB-MSCs detected by immunostaining with anti-human specific RNP antibody. Most implants contained heterogeneous tissues generated by cells as hES cells, however, no teratoma occurred (FIG. 9).

**The phenotype of the hUCB cells that adhered to and subsequently cultured on the ECM is different from that of hES and hBM-MSCs.** In order to further define and characterize the hUCB-MSCs, the inventors examined whether UCB-MSCs isolated by ECM adhesion expressed *NANOG, OCT4, TDGF1, DNMT3B, GABRB3* and *Sox2,* all of which are strongly expressed by hES cells (Adewumi *et al.,* 2007). It was found that the levels of these genes expressed by UCB-MSCs isolated by ECM adhesion were much lower than those expressed by hES cells, but still higher than the levels expressed by both UCB-MSCs isolated by plastic adhesion as well as by human bone marrow-derived mesenchymal stem cells (hBM-MSCs) (FIG. 10). This may explain why UCB-MSCs isolated by ECM adhesion did not form teratoma, as previous studies have clearly shown that somatic cells incorporated with some of these genes do form teratoma. It appears that UCB may contain a large number of embryonic-like stem cells that adhere to BM-derived ECM, but not to plastic and that UCB-MSCs obtained by the ECM adhesion method may have properties distinct from those isolated by the classic plastic adhesion method. The differentiation stage of UCB-MSCs obtained by ECM adhesion may be close to ES cells, and earlier in differentiation compared to UCB-MSCs isolated by the traditional plastic adhesion method. Taken together, these data suggest that hUCB-MSCs isolated by ECM adhesion have properties unique enough compared to the previously characterized types of stem cells (hESCs, hBM-MSCs, adipose tissue-MSCs, periosteum-MSCs, *etc.* and even hUCB-MSCs isolated using the standard plastic adhesion method) to warrant the placement of these cells into a different class of stem cell.

All of the compositions and/or methods disclosed and claimed herein can be made and executed without undue experimentation in light of the present disclosure. While the compositions and methods of this invention have been described in terms of some embodiments, it will be apparent to those of skill in the art that variations may be applied to the compositions and methods and in the steps or in the sequence of steps of the method described herein without departing from the concept, and spirit of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, and concept of the invention as defined by the appended claims.

### REFERENCES

The following references, to the extent that they provide exemplary procedural or other details supplementary to those set forth herein.
Adewumi,O et al., Nat. Biotechnol. 25:803-816, 2007
Chen et al., J. Bone Miner. Res., 22:1943-1956, 2007
Kern et al., Stem Cells, 24:1294-1301, 2006
Soleimani and Nadri, Nat. Protoc. 4:102-106, 2009
Wolfe et al., Methods Mol. Biol., 449:3-25, 2008

## Claims

1. A method of isolating mesenchymal stem cells (MSCs) comprising:
a. seeding a sample on an extracellular matrix (ECM)-precoated culture dish, wherein the sample contains mononuclear cells derived from umbilical cord blood (UCB) and wherein the ECM is formed by culturing bone marrow cells on the culture dish; and
b. isolating the MSCs from the sample.

2. The method of claim **1**, wherein the umbilical cord blood is human UCB (hUCB).

3. The method of claim **1** or **2**, wherein the UCB is collected after birth.

4. The method of any one of claims **1** to **3**, wherein the UCB sample is centrifuged to collect mononuclear cells before seeding.

5. The method of any one of claims **1** to **4**, wherein the ECM is derived from human bone marrow cells.

6. The method of any one of claims **1** to **5**, wherein the ECM comprises collagen type I, collagen type III, fibronectin, biglycan, decorin, perlecan, and/or laminin.

7. A method of promoting MSCs proliferation comprising:
a. obtaining isolated MSCs by a method according to any one of claims **1** to **6**; and
b. seeding the isolated MSCs onto an ECM to promote proliferation of the isolated MSCs.

8. The method of claim **7**, wherein the ECM comprises collagen type I, collagen type III, fibronectin, biglycan, decorin, perlecan, and/or laminin.

## Patentansprüche

1. Verfahren zum Isolieren von mesenchymalen Stammzellen (MSCs), umfassend:
a. impfen einer Probe auf einer Extrazellularmatrix (ECM)-vorbeschichteten Kulturschale, wobei die Probe mononukleare Zellen enthält, die von Nabelschnurblut (UCB) abgeleitet sind, und wobei die ECM durch Kultivieren von Knochenmarkzellen auf der Kulturschale gebildet wird; und
b. isolieren der MSCs aus der Probe.

2. Verfahren nach Anspruch **1**, wobei das Nabelschnurblut menschliches UCB ist (hUCB).

3. Verfahren nach Anspruch **1** oder **2**, wobei das UCB nach der Geburt gesammelt wird.

4. Verfahren nach einem der Ansprüche **1** bis **3**, wobei die UCB-Probe zentrifugiert wird, um mononukleare Zellen vor der Impfung zu sammeln.

5. Verfahren nach einem der Ansprüche **1** bis **4**, wobei die ECM aus menschlichen Knochenmarkzellen abgeleitet ist.

6. Verfahren nach einem der Ansprüche **1** bis **5**, wobei die ECM Collagen Typ I, Collagen Typ III, Fibronectin, Biglycan, Decorin, Perlecan und/oder Laminin umfasst.

7. Verfahren zur Förderung der Proliferation von MSCs, umfassend:
a. erhalten von isolierten MSCs durch ein Verfahren nach einem der Ansprüche **1** bis **6**; und
b. impfen der isolierten MSCs auf ein ECM, um die Proliferation der isolierten MSCs zu fördern.

8. Verfahren nach Anspruch **7**, wobei die ECM Collagen Typ I, Collagen Typ III, Fibronectin, Biglycan, Decorin, Perlecan und/oder Laminin umfasst.

## Revendications

1. Une méthode d'isolement de cellules souches mésenchymateuses (CSMs) comprenant :
a. ensemencer un échantillon sur une boîte de culture pré-recouverte d'une matrice extracellulaire (MEC), dans lequel l'échantillon contient des cellules mononucléaires dérivées du sang du cordon ombilical (SCO) et dans lequel la MEC est formée en cultivant des cellules de moelle osseuse sur la boîte de culture ; et
b. isoler les CSMs de l'échantillon.

2. La méthode selon la revendication **1**, dans laquelle le sang du cordon ombilical est du SCO humain (SCOh).

3. La méthode selon la revendication **1** ou **2**, dans laquelle le SCO est collecté après naissance.

4. La méthode selon l'une quelconque des revendications **1** à **3**, dans laquelle l'échantillon de SCO est centrifugé pour collecter des cellules mononucléaires avant l'ensemencement.

5. La méthode selon l'une quelconque des revendications **1** à **4**, dans laquelle la MEC est dérivée de cellules de moelle osseuse humaine.

6. La méthode selon l'une quelconque des revendications **1** à **5**, dans laquelle la MEC comprend du collagène de type I, du collagène de type III, de la fibronectine, du biglycane, de la décorine, du perlecan, et/ou de la laminine.

7. Une méthode pour promouvoir la prolifération des CSMs comprenant :
a. obtenir des CSMs isolées par une méthode selon l'une quelconque des revendications **1** à **6** ; et
b. ensemencer les CSMs isolées sur une MEC pour promouvoir la prolifération des CSMs isolées.

8. La méthode selon la revendication **7**, dans laquelle la MEC comprend du collagène de type I, du collagène de type III, de la fibronectine, du biglycane, de la décorine, du perlecan, et/ou de la laminine.
